Europäisches Patentamt

European Patent Office

Office européen des brevets

(19)

(11) Veröffentlichungsnummer: **0 382 020**
**A2**

(12) **EUROPÄISCHE PATENTANMELDUNG**

(21) Anmeldenummer: **90101571.9**

(22) Anmeldetag: **26.01.90**

(51) Int. Cl.5: **A61K 7/28**

(30) Priorität: **04.02.89 DE 3903348**

(43) Veröffentlichungstag der Anmeldung:
**16.08.90 Patentblatt 90/33**

(84) Benannte Vertragsstaaten:
**GR**

(71) Anmelder: **Henkel Kommanditgesellschaft auf Aktien**
**Henkelstrasse 67**
**D-4000 Düsseldorf 13(DE)**

(72) Erfinder: **Klüppel, Hans-Jürgen, Dr.**
**Begonienstrasse 7**
**D-4000 Düsseldorf(DE)**
Erfinder: **Förg, Franz, Dr.**
**Rotdornweg 10**
**D-4018 Langenfeld(DE)**

(54) **Mund- und Zahnpflegemittel mit polysaccharidspaltenden Enzymen.**

(57) Mund- und Zahnpflegemittel mit polysaccharidspaltenden Enzymen, insbesondere Dextronase, enthalten als enzymverträgliche und gut schäumende Tenside mit guten Benetzungseigenschaften Alkylglykoside, bevorzugt solche der allgemeinen Formel
RO-(C$_6$H$_{10}$O$_5$)$_x$-H
worin R den aliphatischen Rest eines primären Fettalkohols mit 8 bis 22 C-Atomen und -(C$_6$H$_{10}$O$_5$)$_x$-H einen Oligoglycosidrest mit einem mittleren Oligomerisationsgrad x von 1 - 10, bevorzugt von 1 - 3, darstellt.

EP 0 382 020 A2

## Mund- und Zahnpflegemittel mit polysaccharidspaltenden Enzymen

Die Erfindung betrifft Mund- und Zahnpflegemittel mit einem Gehalt an polysaccharidspaltenden Enzymen und Tensiden, welche diese Enzyme nicht schädigen.

Zur Pflege der Zähne, des Zahnfleisches und der Mundhöhle werden vor allem Zahnpasten, Zahnpulver und Mundwässer verwendet. Diese Produkte enthalten üblicherweise Tenside, um die Reinigungswirkung der Zahnbürste und der in Zahnpasten und Zahnpulvern enthaltenen Abrasivstoffe zu unterstützen. Daneben enthalten Mund- und Zahnpflegemittel meist Wirkstoffe zur Härtung des Zahnschmelzes sowie zur Bekämpfung von Zahnstein und bakteriellen Belägen (Plaque). Es ist bekannt, daß Dextrane, die durch bakterielle Polymerisation aus Glucose gebildet werden, eine wichtige Rolle bei der Bildung der Zahnplaque spielen und man hat daher mehrfach vorgeschlagen, Mund- und Zahnpflegemitteln Enzyme mit polysaccharidspaltender Wirkung, insbesondere Dextranase, beizufügen, um den Bildungsmechanismus der Zahnplaque zu stören oder frisch gebildete Beläge wieder zu lösen (vgl. JADA, Vol. 82, Jan. 1971, 123-141, GB-1202629).

Es ist auch bekannt, daß viele oberflächenaktive Stoffe die Wirkung von Enzymen hemmen und es wurden daher bereits spezielle, mit Enzymen besser verträgliche Tenside zum Einsatz in Dextranase enthaltende Mund- und Zahnpflegemittel vorgeschlagen. Gemäß GB-1.319.423 soll das N-Lauroyloxyethyl-Sulfoacetamid-Kaliumsalz besonders verträglich mit Dextranase sein. Zahlreiche andere Patentanmeldungen schlagen stabilisierende Zusätze vor, um die Dextranase vor der Schädigung durch Tenside zu bewahren.

Die bisher vorgeschlagenen Tenside haben, soweit sie überhaupt mit Dextranase eine bessere Verträglichkeit aufweisen, erhebliche anwendungstechnische Nachteile, da meistens das Schaumverhalten unbefriedigend oder die Benetzungseigenschaften nicht ausreichend sind. Diese Nachteile werden durch die erfindungsgemäßen Mund- und Zahnpflegemittel weitgehend behoben.

Gegenstand der Erfindung sind Mund- und Zahnpflegemittel mit einem Gehalt an (A) polysaccharidspaltenden Enzymen und (B) oberflächenaktiven Stoffen in einem wäßrigen Träger, dadurch gekennzeichnet, daß als oberflächenaktive Stoffe Alkylglycoside enthalten sind.

Unter Mund- und Zahnpflegemitteln im Sinne der Erfindung werden in erster Linie Zahnpasten und Mundwässer verstanden, die einen wäßrigen Träger enthalten. Es können jedoch auch Zahnpulver zu den erfindungsgemäßen Zübereitungen gerechnet werden, da diese regelmäßig mit Wasser zur Anwendung kommen und bei der Anwendung daher ebenfalls Wasser als Trägerkomponente benötigen.

Als polysaccharidspaltende Enzyme werden alle Carbohydrasen, die eine enzymatische, hydrolytische Spaltung von Kohlenhydratbestandteilen katalysieren, verstanden. Solche Enzyme werden nach bekannten Verfahren aus Mikroorganismen gewonnen. Beispiele für geeignete Enzyme sind z.B. $\alpha$- und $\beta$-Glucosidasen, $\alpha$-und $\beta$-Galactosidase, $\alpha$ und $\beta$-Fructosidase, $\alpha$- und $\beta$-Amylase, Glucoamylase ($\alpha$-1,4-Glucanglucohydrolase), Cellulase, Saccharase, Mutanase und Dextranase. Besonders bevorzugt ist die Verwendung von Detranase (1,6-$\alpha$-D-Glucan-6-glucanohydrolase) und/oder 1,3-Glucanase als polysaccharidspaltendes Enzym.

Alkylglycoside, ihre Herstellung und ihre Verwendung als oberflächenaktive Stoffe sind beispielsweise aus US-A-3 839 318, US-A-3 707 535, US-A-3 547 828, DE-A-1 943 689, DE-A-2 036 472 und DE-A-3 001 064 sowie EP-A-77167 bekannt. Ihre Herstellung erfolgt insbesondere durch Umsetzung von Glucose oder Oligosacchariden mit primären Alkoholen mit 8-22 C-Atomen. Bezüglich des Glycosidrestes gilt, daß sowohl Monoglyoside, bei denen ein cyclischer Zuckerrest glycosidisch an den Fettalkohol gebunden ist, als auch oligomere Glycoside mit einem Oligomerisationsgrad bis vorzugsweise 10 geeignet sind. Bevorzugt geeignete Alkylglycoside für die Herstellung der erfindungsgemäßen Zubereitungen sind solche der allgemeinen Formel $RO-(C_6H_{10}O_5)_x-H$, worin R einen aliphatischen Rest eines primären Fettalkohols mit 8 - 22 C-Atomen und - $(C_6H_{10}O_5)_xH$ ein Oligoglucosid-Rest mit einem mittleren Oligomerisationsgrad x von 1 - 10 ist. Speziell bevorzugt sind Oligoglucoside der vorgenannten allgemeinen Formel, worin R einen aliphatischen, linearen Alkylrest mit 10-16 C-Atomen und-$(C_6H_{10}O_5)_x-H$ einen Oligoglucosidrest mit einem mittleren Oligomerisationsgrad x von 1 - 3 bedeutet. Der mittlere Oligomerisationsgrad ergibt sich aus den molaren Anteilen der einzelnen Oligomeren durch Division der Summe der Struktureinheiten durch die Summe der Moleküle (vgl. Principles of Polymer Chemistry, Paul J. Flory, Cornell University Press, Ithaca, New York 1953, Seite 36-37).

In den erfindungsgemäßen Mund- und Zahnpflegemitteln sind die polysaccharidspaltenden Enzyme in Mengen von 100 - 20.000 Einheiten pro g des Zahnpflegemittelns enthalten. Eine Einheit entspricht dabei der Menge an Enzym, die bei Inkubation mit einem spaltbaren Polysaccharid, z.B. mit Dextran, die Bildung von 1 $\mu$g Glucose pro Minute bewirkt. Bevorzugt sind die polysaccharidspaltenden Enzyme in solchen Mengen in den Mund- und Zahnpflegemitteln enthalten, daß bei einer einzelnen Anwendung etwa 2.500 - 30.000 Einheiten des Enzyms in die Mundhöhle gelangen. Bei Verwendung eines Dextranase-Präparats mit einem Gehalt von 200.000 -2.000.000 Einheiten pro g bedeutet dies eine Zusatzmenge von 0,05 - 3,0 Gew.-% zu Zahnpasten und Mundwässern.

Die Alkylglycoside sind bevorzugt in einer Menge von 0,1 - 5 Gew.-% in den erfindungsgemäßen Mund- und Zahnpflegemitteln enthalten. Bei Verwendung der üblichen, ca. 50 %igen wäßrigen Alkylglucosid-Zubereitungen müssen daher etwa 0,2 - 10 Gew.-% dieser Alkylglucosid-Zubereitungen eingesetzt werden. In Mundwasser-Konzentraten, die vor der Anwendung verdünnt werden, sind dem vorgesehenen Verdünnungsverhältnis entsprechend höhere Konzentrationen einzusetzen.

Neben den kennzeichnenden, polysaccharidspaltenden Enzymen und den Alkylglycosiden enthalten die erfindungsgemäßen Mund- und Zahnpflegemittel die für die jeweilige Zubereitungsform üblichen wäßrigen Träger.

Bei den Mundwässern besteht der Träger üblicherweise aus einer wäßrigen oder wäßrig-ethanolischen Lösung von äetherischen Ölen, adstringierenden und tonisierenden Drogenauszügen, karieshemmenden Zusätzen, z.B. Natriumfluorid oder Monofluorphosphat, zahnstein hemmenden Zusätzen, z.B. Organophosphonaten wie 1-Hydroxyethan-1,1-diphosphonsäure, Phosphonopropan-1,1,3-tricarbonsäure oder 1-Azacycloheptan-1,1-diphosphonsäure oder deren wasserlöslichen Salzen und ggf. Süßstoffen.

Bei den Zahnpasten oder Zahncremes besteht der Träger im allgemeinen aus pastösen Zubereitungen aus Wasser, Konsistenzreglern, Feuchthaltemitteln, Schleif- oder Putzkörpern, Süßungsmitteln, Aromastoffen, deodorierenden Wirkstoffen sowie Wirkstoffen gegen Mund- und Zahnerkrankungen. In die erfindungsgemäßen Zahnpasten können alle üblichen Putzkörper wie z.B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogel-Kieselsäuren, Hydrogelkieselsäuren, Fällungskieselsäuren, Aluminiumoxid-trihydrat und feinteiliges α-Aluminiumoxid oder Mischungen dieser Putzkörper in Mengen von 15 - 40 Gew.-% der Zahnpaste.

Als Feuchthaltemittel können z.B. Glycerin, Sorbit, Xylit, Propylenglycole, Polyethylenglycole, insbesondere solche mit mittleren Molekulargewichten von 200 - 800 eingesetzt werden. Als Konsistenzregler (bzw. Bindemittel) dienen z.B. natürliche und/oder synthetische wasserlösliche Polymere wie Carragheenate, Traganth, Stärke und Stärkeether, Celluloseether wie z .B. Carboxymethylcellulose (Na-Salz), Hydroxyethylcellulose, Methylhydroxypropylcellulose, Guar, Akaziengum, Agar-Agar, Xanthangum, Johannisbrotmehl, Pectine, wasserlösliche Carboxyvinylpolymere (z.B. Carbopol[R]-Typen), Polyvinylalkohol, Polyvinylpyrrolidon, Polyethylenglycol, insbesondere solche mit Molekulargewichten von 1 500 -1 000 000.

Weitere Stoffe, die sich zur Viskositätskontrolle eignen, sind z.B. Schichtsilikate wie z.B. Montmorillonit-Tone, kolloidale Verdickungskieselsäure wie z.B. Aerogel-Kieselsäure oder pyrogene Kieselsäuren. Ein für die Herstellung von Zahnpasten mit der erfindungsgemäßen Enzym-Alkylglycosid-Kombination besonders gut geeigneter Träger enthält z.B.

20 - 35 Gew.-% Wasser

20 - 35 Gew.-% Sorbit

5 - 15 Gew.-% Glycerin

2 - 10 Gew.-% Polyethylenglycol (mittleres Molekulargewicht 200 - 800)

0,1-0,5 Gew.-% Carboxymethylcellulose

1 - 3 Gew.-% Verdickungskieselsäure und

14 - 40 Gew.-% Schleif- und Putzkörper.

Weitere übliche Zahnpastenzusätze sind

- Konservierungsmittel und antimikrobielle Stoffe, wie z.B. p-Hydroxybenzoesäuremethyl-, -ethyl oder propylester, Natriumsorbat, Natriumbenzoat, Bromchlorophen, Phenyl-salicylsäureester, Thymol usw. Antizahnsteinwirkstoffe, z. B. Organophosphonate wie die die Natriumsalze von 1-Hydroxyethan-1,1-diphosphonsäure, Azacycloheptan 1-Phosphonopropan-1,2,3-tricarbonsäure und andere Phosphonsäuren wie sie z.B. aus US-PS 3 488 419, DE-OS 22 24 430 und DE-OS 23 43 196 bekannt sind,

- Karieshemmende Stoffe wie z.B. Natriumfluorid, Natriummonofluorphosphat, Zinnfluorid

- Süßungsmittel wie z.B. Saccharin-Natrium, Natrium-Cyclamat, Sucrose, Lactose, Maltose, Fructose,

- Aromen wie z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen,

- Pigmente wie z.B. Titandioxid

- Farbstoffe

- Puffersubstanzen wie z.B. primäre, sekundäre oder tertiäre Alkaliphosphate oder Citronensäure/Natriumcitrat,

3

- Wundheilende und entzündungshemmende Stoffe wie z.B. Allantoin, Harnstoff, Azulen, Kamillenwirkstoffe oder Acetylsalicylsäurederivate.

Die erfindungsgemäß zu verwendenden Alkylglycoside sind vorzugsweise die einzigen, in den Mund- und Zahnpflegemitteln enthaltenen oberflächenaktiven Stoffe. Allenfalls können gewisse nichtionische Tenside enthalten sein, die ebenfalls nur eine geringe Schädigung der polysaccharidabbauenden Enzyme bewirken. Solche Tenside, die ggf. zur Solubilisierung von Aromakomponenten nützlich sein könnten, sind z.B. die oxethylierten Fettsäureglyceride, die oxethylierten Fettsäuresorbitanpartialester oder die Fettsäurepartialester von Glycerin- oder Sorbitan-oxethylaten. Solche lösungsvermittelnde nichtionische Tenside können ggf. neben den Alkylglycosiden in einer Menge von 0,01 - 0,3 Gew.-% bezogen auf das gesamte Mittel in dem Träger enthalten sein.

Die folgenden Beispiele sollen den Erfindungsgegenstand näher erläutern:

## Beispiele

### 1. Prüfung der Verträglichkeit zwischen Tensiden und polysaccharidspaltenden Enzymen.

#### 1.1 Meßprinzip

Die Aktivität und der Aktivitätsverlust polysaccharidspaltender Enzyme unter dem Einfluß von Tensiden wurde am Beispiel von 1,6-$\alpha$-D-Glucan-6-Glucanohydrolase dadurch bestimmt, daß die Freisetzung von Glucose aus einer Dextranlösung unter dem Einfluß des Enzyms und von Tensiden über definierte Zeiträume gemessen wurde. Die Menge der freigesetzten Glucose wurde mit Hilfe von 3,5-Dinitrosalicylsäure (die durch die Aldehydfunktion der Glucose zu einer orangerot gefärbten Verbindung (wahrscheinlich 3-Amino-5-nitrosalicylsäure) reduziert wird) spektrometrisch bestimmt.

#### 1.2 Durchführung

1 Portion Dextranase (1,6-$\alpha$-D-Glucan-6-Glucanohydrolase; Enzymkatalog EC 3.2.1.11, Fa. Sigma), Bestr. Nr. D 1508, 100 Units, wurde in 1 ml Phosphatpuffer-Lösung (PH = 7) gelöst. 10 $\mu$l dieser Lösung wurden mit 0,5 ml der Tensidlösung (2 Gew.-% Tensid in Wasser) gemischt und über den Prüfzeitraum gelagert.

Aktivitätsbestimmung: Nach 5 Minuten Temperierung im Wasserbad bei 35 °C wurde 1 ml einer 1 %igen Lösung von Dextran (Fa. Sigma, D 5376, Molmasse 2.000.000) zugegeben. Nach 15 Minuten bei 35 °C wurden 2 ml "DNS-Reagens" (Zusammensetzung siehe unter 1.3) zugegeben.

Dann wurde die Lösung 15 Minuten auf 100 °C erhitzt, 2 Minuten unter fließendem Wasser auf ca. 20 °C abgekühlt und mit destilliertem Wasser auf 10 ml ergänzt. Nach weiteren 30 Minuten wurde die Absorption bei einer Wellenlänge von 530 nm gemessen. Aus der Eichkurve, die nach Reaktion von Glucose-Lösungen bekannter Konzentration mit "DNS-Reagenz" aufgenommen worden war, konnte die Menge an freigesetzter Glucose - und damit die Aktivität des Enzyms - ermittelt werden.

#### 1.3 Zusammensetzung des "DNS-Reagenz"

15 g 3,5-Dinitrosalicylsäure (DNS) wurden in 800 ml enthärtetem Wasser gelöst und mit 300 ml 4,5 %iger Natriumhydroxidlösung versetze. In dieser Lösung wurden 225 g Kalium-Natrium-tartrat aufgelöst. Anschließend wurde mit 69 g Phenollösung (10 g Phenol und 22 ml 10 %ige Natriumhydroxidlösung auf 100 ml mit enthärtetem Wasser ergänzt) und 6,9 g Natriumhydrogencarbonat versetzt. Die so erhaltene Lösung wurde 48 Stunden stehengelassen, dann filtriert und im Dunkeln aufbewahrt.

#### 1.4 Ergebnis

Für die Tenside

(1) Na-Laurylsulfat

(2) Cetyl-Stearylalkohol (30:70)polyglykolether (25 Mol EO)

(3) Di-Na-Sulfobernsteinsäure-monolaurat

(4) Proteinhydrolysat-Fettsäurekondensationsprodukt (Na-Salz) und

(5) Alkyl (C 12/C 14 = 70 : 30)-oligo (x = 1,2)-Glucosid wurden die folgenden Enzymaktivitäten nach 1 Stunde, 1 Woche und 4 Wochen ermittelt:

| Tensid | 1 Stunde | 1 Woche | 4 Wochen |
|---|---|---|---|
| Standard (tensidfrei) | 640 | 570 | 560 |
| (1) | 230 | 0 | 0 |
| (2) | 575 | 620 | 285 |
| (3) | 525 | 415 | 250 |
| (4) | 790 | 620 | 330 |
| (5), Erfindung | 610 | 675 | 500 |

2. Rezeptur-Beispiele

| Zahncreme | Angaben in Gew.-% |
|---|---|
| Na-Carboxymethylcellulose | 1,10 % |
| Glycerin, 86 % | 10,00 % |
| Sorbit, 70 % | 15,00 % |
| Saccharin-Natrium | 0,05 % |
| Natriumcyclamat | 0,10 % |
| Natriummonofluorphosphat | 1,14 % |
| PHB-Methylester | 0,15 % |
| Aroma | 1,00 % |
| Calciumhydrogenphosphat-Dihydrat | 25,00 % |
| Calciumhydrogenphosphat, wasserfrei | 7,00 % |
| Siliciumdioxid, hochdispers | 2,00 % |
| Alkylglucosid | 1,50 % |
| Dextranase (1.000.000 Einheiten pro g) | 0,45 % |
| Wasser | ad 100 % |

| Gel-Zahncreme | |
|---|---|
| Na-Carboxymethylcellulose | 0,40 % |
| Sorbit, 70 % | 72,00 % |
| Polyethylenglykol 1500 | 3,00 % |
| Saccharin-Natrium | 0,07 % |
| Natriumfluorid | 0,24 % |
| PHB-Ethylester | 0,15 % |
| Aroma | 1,10 % |
| Abrasivkieselsäure | 11,00 % |
| Verdickungskieselsäure | 6,00 % |
| Alkylglucosid | 1,40 % |
| Dextranase (1.000.000 Einheiten pro g) | 0,50 % |
| Wasser | ad 100 % |

| Zahnpulver | |
|---|---|
| Na-Carboxymethylcellulose | 1,50 % |
| Polyethylenglykol 1500 | 4,00 % |
| Saccharin-Natrium | 0,30 % |
| Aroma | 1,40 % |
| Siliciumdioxid, hochdispers | 3,50 % |
| Calciumcarbonat, praecipit. | 77,50 % |
| Alkylglucosid | 1,40 % |
| Dextranase (1.000.000 Einheiten pro g) | 0.40 % |

| Mundwasser | |
|---|---|
| Ethanol, 96 Vol. % unvergällt | 5,00 % |
| Sorbit, 70 % | 3,00 % |
| Sacharin-Natrium | 0,07 % |
| PHB-Methylester | 0,10 % |
| Aroma | 0,15 % |
| Alkylglucosid | 0,70 % |
| Dextranase (1.000.000 Einheiten pro g) | 0,12 % |
| Wasser | ad 100 % |

**Ansprüche**

1. Mund- und Zahnpflegemittel mit einem Gehalt an (A) polysaccharidspaltenden Enzymen und (B) oberflächenaktiven Stoffen in einem wäßrigen Träger, dadurch gekennzeichnet, daß als oberflächenaktive Stoffe Alkylglycoside enthalten sind.

2. Mund- und Zahnpflegemittel nach Anspruch 1, dadurch gekennzeichnet, daß als polysaccharidspaltende Enzyme Dextranase und/oder 1,3-Glucanase enthalten sind.

3. Mund- und Zahnpflegemittel nach Anspruch 1 oder 2, dadurch gekennzeichnet, daß als oberflächenaktive Alkylglycoside solche der allgemeinen Formel $RO-(C_6H_{10}O_5)_x-H$ enthalten sind, worin R einen aliphatishen Rest eines primären Fettalkohols mit 8 bis 22 C-Atomen und $-(C_6H_{10}O_5)_x-H$ ein Oligoglycosidrest mit einem mittleren Oligomerisationsgrad x von 1 - 10 ist.

4. Mund- und Zahnpflegemittel nach Anspruch 3 dadurch gekennzeichnet, daß R einen aliphatischen linearen Alkylrest mit 10 - 16 C-Atomen und $-(C_6H_{10}O_5)_xH$ ein Oligoglucosid-Rest mit einem mittleren Oligomerisationsgrad x = 1-3 ist.

5. Mund- oder Zahnpflegemittel nach Anspruch 1-4, dadurch gekennzeichnet, daß der wäßrige Träger ein Mundwasser in Form einer wäßrigen oder wäßrig-ethanolischen Lösung mit Geschmacks- und Aromazusätzen oder eine Zahnpasta in Form einer wäßrigen Dispersion von Schleif- und Putzkörpern mit Verdickungsmitteln, Feuchthaltemilteln, Geschmacks- und Aromazusätzen ist.